(19) Europäisches Patentamt
European Patent Office
Office européen des brevets

(11) Veröffentlichungsnummer: **0 036 433**
**B1**

(12) # EUROPÄISCHE PATENTSCHRIFT

(45) Veröffentlichungstag der Patentschrift: **20.02.85**

(21) Anmeldenummer: **80101478.8**

(22) Anmeldetag: **20.03.80**

(51) Int. Cl.$^4$: **C 07 D 307/60,**
C 07 D 303/14,
C 07 D 307/20, C 07 D 307/32
// A23L 1/235

(54) **Verfahren zur Herstellung von 2,5-Dimethyl-4-hydroxy-2,3-dihydrofuran-3-on und Zwischenprodukte.**

(43) Veröffentlichungstag der Anmeldung:
**30.09.81 Patentblatt 81/39**

(45) Bekanntmachung des Hinweises auf die Patenterteilung:
**20.02.85 Patentblatt 85/08**

(84) Benannte Vertragsstaaten:
**CH DE FR NL**

(56) Entgegenhaltungen:
**EP-A-0 008 358**
**DE-A-1 768 649**
**DE-A-2 359 891**
**DE-A-2 835 368**

**Chemical Abstracts Band 90, 30. Juni 1979 Columbus, Ohio, USA FORMULA INDEX, "3(2H)-Furanone, dihydro-4-hydroxy-2,5-dimethyl- (70097-95-9), P 186771p" Seite 295F, Spalte 3**

**THE JOURNAL OF ORGANIC CHEMISTRY, Band 43, Nr. 25, 8. Dezember 1978, Easton G. BÜCHI et al. "Synthesis of beta-lains" Seiten 4765 bis 4769**

(73) Patentinhaber: **BASF Aktiengesellschaft**
**Carl-Bosch-Strasse 38**
**D-6700 Ludwigshafen (DE)**

(72) Erfinder: **Ross, Karl-Heinz, Dr.**
**Sudetenstrasse 8**
**D-6704 Mutterstadt (DE)**
Erfinder: **Dudeck, Christian, Dr.**
**Odenwaldring 20**
**D-6703 Limburgerhof (DE)**
Erfinder: **Himmele, Walter, Dr.**
**Eichenweg 14**
**D-6909 Walldorf (DE)**
Erfinder: **Lebkuecher, Rolf, Dr.**
**Kranichstrasse 9**
**D-6700 Ludwigshafen (DE)**
Erfinder: **Sauer, Wolfgang, Dr.**
**Langstrasse 23**
**D-6719 Kirchheimbolanden (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 036 433 B1

**Beschreibung**

Die vorliegende Erfindung betrifft ein neues Verfahren zur Herstellung von 2,5-Dimethyl-4-hydroxy-2,3-dihydrofuran-3-on (I)

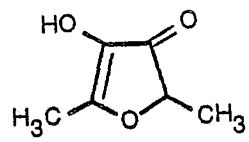

I,

sowie die neuen Vorstufen dieser Verbindung, nämlich 2,5-Dimethyl-3,4-dihydroxy-tetrahydrofuran (III) und 2,5-Dimethyl-4-hydroxy-tetrahydrofuran-3-on (IV).

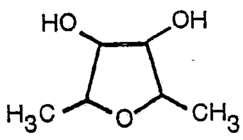

III

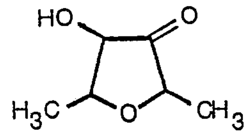

IV

Im Journal of Org. Chem. *43* Nr. 25 (1978), Seite 4767, Spalte 2, Zeile 24 wird zwar "cis 3,4-Dihydroxy-2,5-*dimethyl*-tetrahydrofuran" bereits als Ausgangsmaterial zur Herstellung von "meso-tartaric dialdehyd" genannt, jedoch geht aus der dort beschriebenen Reaktion sowie der diesbezüglich zitierten Originalliteratur klar hervor, daß es sich hierbei um einen Druckfehler handelt und es richtig "cis-3,4-Dihydroxy-2,5-di*methyloxy*-tetrahydrofuran" heißen müßte.

Die Synthese des natürlichen, in Ananas und Erdbeeren vorkommenden Fruchtaromastoffes I ist Gegenstand zahlreicher Verfahrensvorschriften, die jedoch aus verschiedenen Gründen unbefriedigend sind und sich auf wirtschaftliche Weise nicht in den technischen Maßstab übertragen lassen.

So geht man beispielsweise nach der Arbeit von Büchi und Demole (J. Org. Chem., Band 38, 1973, Seite 123f) von 2,5-Dimethyl-furanon aus, überführt dieses durch Bromierung in methanolischer Lösung in das 2,5-Dimethyl-2,5-dimethoxy-2,5-dihydrofuran, oxidiert die letztgenannte Verbindung mit Kaliumchlorat und katalytischen Mengen Osmiumtetroxid zum 3,4-Dihydroxy-hexan-2,5-dion, welches sich in Gegenwart von Basen zu (I) cyclisieren läbt. Diese Synthese ist umständlich und erfordert die Verwendung des nicht unproblematischen Kaliumchlorates sowie des teuren und stark giftigen Osmiumtetroxides, so daß sie für technische Zwecke nicht in Betracht kommt.

Ebenso unbefriedigend ist eine von den gleichen Autoren (loc. cit.) beschriebene Variante dieses Verfahrens, bei der lediglich das 3,4-Dihydroxy-hexan-2,5-dion auf einem anderen Wege, nämlich durch Hydrodimerisierung von Methylglyoxal in Gegenwart von Zinkstaub hergestellt wird. Diese Reaktion, die aus vielen Teiloperationen besteht, ist verfahrenstechnisch in größeren Ansätzen nur schwer zu beherrschen und liefert überdies nur Ausbeuten von rund 20%.

Auch das Verfahren der DE—PS 21 05 014, nach welchem 2,5-Dihalogen-hexan-3,4-dione mit wäßrigem Alkali zu I cyclisiert werden, läßt zu wünschen übrig, da man hierbei nur Ausbeuten von etwa 30% erzielt.

In einem ähnlichen Verfahren (DE—OS 17 68 649) wird 2,5-Dihydroxy-hexan-3,4-dion zu (I) umgesetzt. Da dieses Dioldion jedoch seinerseits nur durch die gefährliche und technisch schwer zu beherrschende Ozonisierung von 2,5-Dihydroxy-hex-3-in hergestellt werden kann, ist auch hierbei eine technische Realisierung des Verfahrens mit Schwierigkeiten verbunden.

Anstelle der Diol-Dione wurden auch bereits deren Ester wie die Diacetate zur Cyclisierung zu (I) eingesetzt (vgl. DE—OS 19 15 788). Die Herstellung dieser Ester verläuft jedoch über eine Grignard-Reaktion und eine Oxydation mit Kaliumpermanganat, d.h. Verfahrensschritte, die bekanntlich eine exakte Einhaltung ganz bestimmter Reaktionsbedingungen erfordern, wodurch eine technische Synthese schwerfällig und teuer wird.

Bei einem weiteren Verfahren (DT—OS 23 59 891), das über die Kondensation von einem Natriumacetessigester und α-Brompropionylchlorid und anschließende Decarboxylierung und Oxidation verläuft, werden nur Ausbeuten von etwa 15%, bezogen auf das Brompropionylchlorid erzielt.

Aufgabe der Erfindung war es daher, neue Wege und neue Ausgangsverbindungen zu finden, mit deren Hilfe es gelingt, den begehrten Aromastoff (I) auf wirtschaftlichere und verfahrenstechnisch einfachere Weise herzustellen als bisher.

Es wurde nun gefunden, daß man das 2,5-Dimethyl-4-hydroxy-2,3-dihydrofuran-3-on (I) in guten Ausbeuten und in technisch problemlosen Verfahrensschritten auf wirtschaftlichere Weise erhält, wenn man

a) Hex-3-en-2,5-diol (V) in an sich bekannter Weise in flüssiger Phase mit Wasserstoffperoxid in Gegenwart eines Oxids eines Metalles der 4. bis 8. Nebengruppe oder einer Heteropolysäure eines der Säure bildenden Elemente der Gruppe VI des Periodensystems der Elemente bei einem pH-Wert von 3 bis 7, vorzugsweise 4 bis 6, zum 3,4-Epoxyhexan-2,5-diol (II) epoxidiert,

b) das erhaltene neue 3,4-Epoxy-hexan-2,5-diol in an sich bekannter Weise mittels katalytischer Mengen einer Säure unter Spaltung des Epoxidringes und anschließendem Ringschluß in das neue 2,5-Dimethyl-3,4-dihydroxy-tetrahydrofuran (III) überführt,

c) das erhaltene 2,5-Dimethyl-3,4-dihydroxy-tetrahydrofuran mittels Sauerstoff oder einem Sauerstoff enthaltenden Gas bei Temperaturen von 400 bis 700°C an einem Silber- oder Kupfer-Kontakt zu dem neuen 2,5-Dimethyl-4-hydroxy-tetrahydrofuran-3-on (IV) dehydriert und

d) das erhaltene 2,5-Dimethyl-4-hydroxy-tetrahydrofuran-3-on in konzentriert essigsaurer Lösung mit Wismutoxid zu dem 2,5-Dimethyl-4-hydroxy-2,3-dihydrofuran-3-on oxidiert.

Die erfindungsgemäße Synthese läßt sich wie folgt veranschaulichen:

a)

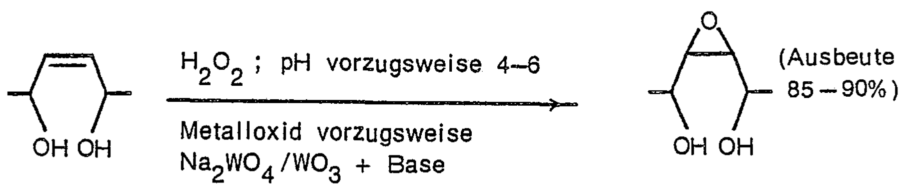


V II

b)

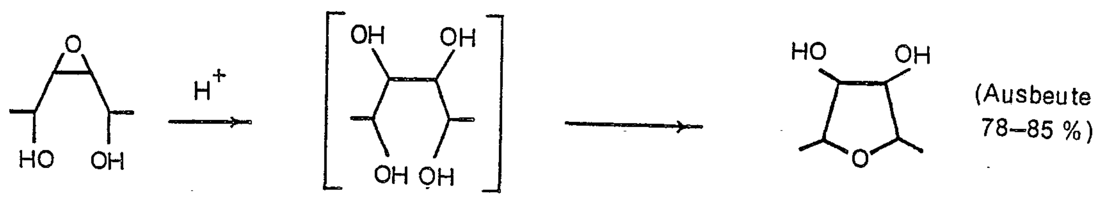


II III

c)

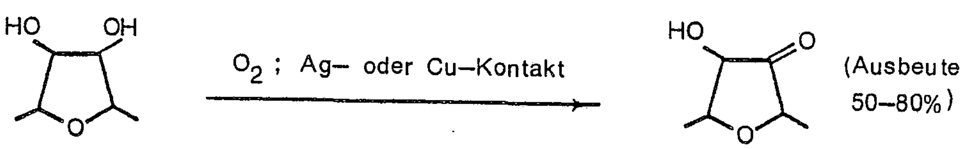


III IV

d)

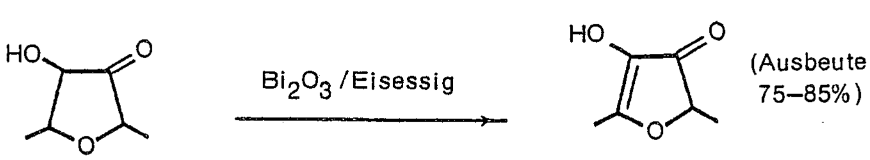


IV I

Die Ausbeuten über die Stufen a) bis d) betragen demnach etwa 30 bis 50%.

Das für den Reaktionsschritt a) benötigte Hex-3-en-2,5-diol ist eine bekannte Verbindung, die z.B. durch partielle Hydrierung des entsprechenden Hexindiols mittels Wasserstoff an einem gemäß der deutschen Patentschrift 11 15 238 mit Zink- oder Bleiionen teilweise desaktivierten Palladiumkontakt in praktisch quantitativer Ausbeute erhältlich ist.

Das Hex-3-in-2,5-diol ist eine handelsübliche Verbindung, die durch Umsetzen von Acetylen mit 2 Mol Acetaldehyd hergestellt werden kann.

Im einzelnen empfiehlt es sich, die Teilschritte a) bis d) des erfindungsgemäßen Verfahrens folgendermaßen auszuführen:

a) Für die Herstellung des 3,4-Epoxy-hexan-2,5-diols arbeitet man im wesentlichen unter den aus der DE—AS 11 44 276 für Epoxidierungen bekannten Reaktionsbedingungen. Man geht zweckmäßigerweise von 20 bis 50 gew.%igen wäßrigen Lösung des Hex-3-en-2,5-diols (V) aus, in der man 1 Gew.%, bezogen auf (V), eines Oxides eines Metalles der 4. bis 8. Nebengruppe des Periodensystems, wie Wolframoxid, Rutheniumtetroxid, Vanadiumtetroxid, Molybdänoxid oder Chromtrioxid, oder aber eine Heteropolysäure eines der säurebildenden Elemente der Gruppe VI des Periodensystems, wie Heteropolywolframsäuren oder auch Molybdowolfram- oder Chromowolframsäuren aufschlämmt. Unter diesen Metalloxiden hat sich Wolframoxid besonders gut bewährt. Diese Suspension stellt man sodann mit einer basischen Verbindung auf einen pH-Wert von 3—7, vorzugsweise 4 bis 6, insbesondere 6 bis 7 ein.

Als basische Verbindungen eignen sich anorganische Basen wie Natriumhydroxid und Natriumcarbonat, besonders jedoch organische Amine, insbesondere wasserlösliche, und zwar im allgemeinen solche mit weniger als 15 Kohlenstoffatomen, nämlich sowohl aliphatische und cycloaliphatische Amine, wie auch aromatische und heterocyclische Amine, wie auch aromatische und heterocyclische, einschließlich primärer, sekundärer und tertiärer Amine. Beispiele für aliphatische Amine sind Methylamin, Dimethylamin, Äthylamin, Diäthylamin, Propylendiamin, Diisopropylamin, Diisobutylamin, Propylendiamin, Diäthylentriamin, Tetraäthylenpentamin, Propanolamin, Propylendiamin, Äthanolamin und Triäthanolamin.

An aromatischen Aminen kommen in Betracht z.B. Anilin, Methylanilin, Dimethylanilin, Toluidin, Xylidin, Diphenylamin und Phenylendiamin. Geeignete heterocyclische Amine sind Pyridin, Pyrrol, Pyrrolidin, Piperidin, Morpholin, N-Methylmorpholin, N-Äthylmorpholin und Lutidin.

Da die Amine die Epoxidierung bereits in geringer Konzentration begünstigen, genügt es, sie in einer Menge von 0,005 bis 2 Gew.%, bezogen auf das Reaktionsgemisch, anzuwenden, und zwar gegebenenfalls gemeinsam mit einer anorganischen Base. Die so erhaltene Suspension wird danach bei 0 bis 100, vorzugsweise 30 bis 60°C allmählich und unter Rühren mit der zu (V) äquimolaren oder leicht überschüssigen Menge Wasserstoffperoxid, zweckmäßigerweise in Form einer 30 bis 50 gew.%- igen wäßrigen Lösung, versetzt. Die Epoxidierung, für die sich um übrigen die Vorschriften der DE—AS 11 44 276 anwenden lassen, nimmt etwa noch 3 bis 12 Stunden in Anspruch. Die destillative Aufarbeitung des noch metalloxidhaltigen Reaktionsgemisches liefert II als Rohprodukt in etwa 85 bis 93 %iger Ausbeute. Das neue 3,4-Epoxyhexan-2,5-diol ist eine viskose Flüssigkeit, die sich bei Destillationsversuchen zersetzt.

b) Es ist ein besonderer Vorteil der erfindungsgemäßen (I) -Synthese, daß die beim Verfahrensschritt a) anfallenden wäßrigen Lösungen unmittelbar für die Folgestufe (b), die Herstellung des 2,5-Dimethyl-3,4-dihydroxytetrahydrofurans (II) eingesetzt werden können.

Hierzu versetzt man die Lösung mit katalytischen Mengen einer Säure und erhitzt das Gemisch etwa 0,5 bis 3 Stunden lang auf 40 bis 180, vorzugsweise 60 bis 120°C. Hierbei bildet sich zunächst das Hexan-2,3,4,5-tetrol, welches nach Abdestillieren des Wassers durch die für eine Destillation notwendige weitere Temperaturerhöhung auf 80 bis 280°C, vorzugsweise 120 bis 180°C, bei 0,2 bis 50 mbar zu (III) dehydratisiert wird.

Als Säuren eignen sich vor allen Mineralsäuren, wie Schwefelsäure, Phosphorsäure und Salzsäure sowie starke organische Säure wie Ameisensäure und Oxalsäure und vor allem p-Toluolsulfonsäure. Verfahrenstechnisch eignen sich auch stark saure Ionenaustauscher. Als stark saure Ionenaustauscher bezeichnen wir im wesentlichen handelsübliche Kationenaustauscher auf der Basis von Polystyrolsulfonsäureharzen oder Phenolsulfonsäureharzen, die Z.B. unter folgendem Handelsnamen bekannt sind: Lewatit S 115®, Amberlite JR 120®, Dowex 50®, Wolfatit KPS 200® und ähnliche.

Zieht man es vor, statt von dem bei der Verfahrensstufe a) anfallenden Reaktionsgemisch von reinem II auszugehen, so verdünnt man dieses zweckmäßigerweise mit der 2 bis 5-fachen Menge Wasser und verfährt im übrigen wie oben angegeben.

Das neue 2,5-Dimethyl-3,4-dihydroxy-tetrahydrofuran ist eine farblose viskose Flüssigkeit, die bei 110°C und 0,3 mbar siedet und nach gaschromatographischer Analyse als Gemisch verschiedener Stereoisomerer vorliegt.

c) Diese Dehydrierungsstufe ist eine heterogene Reaktion an Kupfer- oder vorzugsweise Silber-Festbettkontakten. Diese Reaktion findet unter den Bedingungen der Formaldehyd-Synthese aus Methanol statt, jedoch war es unerwartet, daß III unter den hierbei erforderlichen hohen Temperaturen von 400 bis 700°C, vorzugsweise 480 bis 650°C, insbesondere 510 bis 580°C nicht dehydratisiert. Als Katalysatoren verwendet man vorzugsweise Agglomerate von Silberkristallen mit Teilchendurchmessern von 0,01 bis 2,5 mm. Die Verwendung von Teilchen unterschiedlicher Teilchengröße wird bevorzugt, da sie eine

unregelmäßige Schüttung bewirkt, wodurch die Reaktion mit höherer Selektivität abläuft. Die Schichtdicke des Katalysatorbettes beträgt vorzugsweise 1 bis 3 cm. An 1 kg eines derartigen Kontaktes lassen sich pro Stunde 1 bis 20 kg (III) zu (IV) oxidativ dehydrieren. Zweckmäßigerweise geht man bei der Dehydrierung nicht von einem reinen gasförmigen Gemisch aus (III) und Sauerstoff aus, sondern nimmt die Reaktion in großer Verdünnung vor.

Bewährt hat sich die Verwendung von Luft, die zusätzlich noch mit $N_2$, Wasserdampf oder Acetondampf versetzt wurde. Geeignete gasförmige Ausgangsgemische setzten sich etwa wie folgt zusammen: 5 bis 45 Vol.% (III), 30 bis 85 Vol.% Luft, Rest $N_2$ oder $N_2$/Wasser oder $N_2$/Aceton im Volumenverhältnis von etwa 1:1.

Diese Komponenten werden, nachdem (III) und eventuell weitere normalerweise flüssige Bestandteile verdampft worden sind, möglichst kurz und dann von oben durch das 400 bis 700°C heiße Katalysatorbett geleitet. Die Ausbeute an (IV) ist hierbei umso höher, je schneller man das Gasgemisch nach Passieren des Kontaktes auf möglichst tiefe Temperaturen, z.B. Raumtemperatur abschreckt. Hierzu kann man die Gase mit kaltem Wasser waschen, welches man mit Natronlauge zur Stabilisierung von (IV) zweckmäßigerweise leicht alkalisch, d.h. etwa auf einen pH-Wert von 7 bis 8 einstellt. Die Verweilzeit am Kontakt soll etwa 0,001 bis 0,1 Sekunden betragen.

Aus der Lösung zieht man das Wasser unter vermindertem Druck (etwa 70 bis 13 mbar) ab und fraktioniert den hierbei verbleibenden Rückstand bei etwa 1,3 bis 0,05 mbar. Neben nicht umgesetztem (III) (Sdp. 105 bis 120°C bei 1 mbar) erhält man das 2,5-Dimethyl-4-hydroxy-tetrahydrofuran-3-on, (IV) (Sdp. 61 bis 66°C bei 1 mbar) in bis zu 85 %iger Ausbeute, bezogen auf umgesetztes (III). Der Umsatz beträgt etwa 30 bis 60%. Für die Verwendung von Kupferkontakten gelten die gleichen Verfahrensvorschriften, jedoch ist der Umsatz etwas geringer.

Das neue 2,5-Dimethyl-4-hydroxy-tetrahydrofuran-3-on ist ein interessanter Duft- und Aromastoff, der fruchtig riecht und sich zur Herstellung von kosmetischen Zubereitungen und zur Aromatisierung von Getränken und sonstigen Lebensmitteln eignet. Nach gaschromatographischer Analyse stellt er ein Gemisch verschiedener Stereoisomerer dar.

d) Im Verfahrensschritt d) wird die Oxidation des Ketols (IV) zu (I) nach der Methode von Rigby (J. Chem. Soc. 1951, S. 793f) vorgenommen, indem man zunächst ein Gemisch aus 40 bis 70 Gew.% Eisessig und 25 bis 50 Gew.% Wismutoxid $Bi_2O_3$ herstellt, und dann 10 bis 20 Gew.% (IV) zugibt und 10 bis 30 Minuten lang auf 100 bis 130, vorzugsweise 110 bis 125°C, erhitzt, wobei ein Teil des $Bi_2O_3$ zu metallischem Wismut reduziert wird. Nach dem Abkühlen des Reaktionsgemisches und Abfiltrieren des gebildeten Wismuts arbeitet man das Reakktionsgemisch auf übliche Weise destillativ auf. Zweckmäßig führt man die Destillation bei 0,05 bis 0,5 mbar durch. Anschließende Kristallisation aus Petroläther/Chloroform in bekannter Weise führt zu sehr reinem (I).

## Beispiel

a) Herstellung von 3,4-Epoxy-hexan-2,5-diol.

Eine Suspension aus 696 g Hex-3-en-2,5-diol 90 %iger Reinheit, 7,5 g Wolframtrioxid ($WO_3$), 5,5 g N-Methyl-morpholin und 2 400 g Wasser, wurde innerhalb von 5 Stunden unter Rühren bei 40 bis 50°C allmählich mit 414 g 50 %igem wäßrigem Wasserstoffperoxid (= 1,1 Mol pro Mol Hex-3-en-2,5-diol) versetzt. Das Gemisch wurde noch weitere 17 Stunden bei Raumtemperatur gerührt und anschließend destillativ bei 40°C und 20 mbar vom Wasser befreit. Man erhielt 722 g eines Rückstandes, der nach Epoxidbestimmung zu etwa 85% aus 3,4-Epoxy-hexan-diol bestand. Das entspricht einer Ausbeute von 86% der Theorie.

b) Herstellung von 2,5-Dimethyl-3,4-dihydroxy-tetrahydrofuran.

Die gemäß Verfahrensschritt erhaltene wäßrige Lösung wurde mit 20 g p-Toluolsulfonsäure 2 Stunden lang auf 100°C erhitzt wurde. Nach destillativer Entfernung des Wassers bei Normaldruck wurde der sirupöse, aus dem Hexan-2,3,4,5-tetrol bestehende Rückstand bei 0,5 mbar auf 95 bis 140°C erhitzt, wobei sich das Furan bildete, welches unter diesen Bedingungen abdestillierte. Es wurden 640 g eines Destillats erhalten, das nach gaschromatographischer Analyse zu 88%, aus 2,5-Dimethyl-3,4-dihydroxy-tetrahydrofuran besteht. Das entspricht einer Ausbeute von 79% der Theorie.

Die Feindestillation lieferte das 2,5-Dimethyl-3,4-dihydroxytetrahydrofuran bei 105 bis 112°C und 0,3 mbar in 99 %iger Reinheit. Nach gaschromatographischer Analyse (2 m Carbowachs, 100 bis 180°C) und nach NMR-Spectroskopie lag das Produkt als Gemisch verschiedener Stereoisomeren vor.

c) Herstellung von 2,5-Dimethyl-4-hydroxy-tetrahydrofuran-3-on (IV).

Ein senkrecht stehender Rohrreaktor von 2 cm lichter Weite, an dessen oberen Ende 28 g Silberkristalle (8 g mit Teilchengrößen von 0,75 bis 1 mm Durchmesser, 14 g mit 0,4 bis 0,75 mm Durchmesser und 6 g mit 0,2 bis 0,4 mm Durchmesser) in einer 2 cm starken Schichtung über einem Sieb aus Silbergaze angeordnet waren, wurde stündlich mit einem auf 175°C erhitzten gasförmigen Gemisch aus 18,7 l 2,5-Dimethyl-3,4-dihydroxy-tetrahydrofuran (= 110 g), 72 l Luft und 100 l Stickstoff beschickt. Die Kontakttemperatur betrug 535°C und die Verweilzeit am Kontakt ungefähr 0,04 Sekunden. Nach Passieren des Kontaktes wurde das Gasgemisch sofort auf 20°C abgekühlt, indem man es in mit 10 %iger NaOH auf pH = 8 eingestelltes Wasser einleitete, wobei Stickstoff zurückgehalten wurde. Anschließend wurde das

Wasser bei 20 mbar abgezogen und der Rückstand bei 1,3 mbar destilliert. Hierbei fielen bei 61 bis 66°C 35 g eines Destillats an, das zu etwa 80% aus 2,5-Dimethyl-4-hydroxy-tetrahydrofuran-3-on besteht und bei 109 bis 120°C 60 g an nicht umgesetztem 2,5-Dimethyl-3,4-dihydroxy-tetrahydrofuran. Der Umsatz betrug rund 45 % und die hierauf bezogene Ausbeute etwa 70 %.

d) Herstellung von 2,5-Dimethyl-4-hydroxy-2,3-dihydrofuran-3-on.

17,5 g eines etwa 80 %igen 2,5-Dimethyl-3,4-dihydroxy-tetrahydrofuran-3-ons wurden in einer Suspension aus 40 g Wismutoxid und 50 g Eisessig 25 Minuten auf 120°C erhitzt. Nach dem Abkühlen wurde das Reaktionsgemisch mit 80 g Äthylacetat verdünnt, wobei metallisches Wismut und Wismutacetat ausfielen. Das Filtrat wurde fraktioniert, wobei bei 85 bis 101°C und 0,3 mbar 14,2 g eines Destillats anfiel, das zu rund 77 % aus (I) bestand. Dies entspricht einer Ausbeute von 79 %. Von dieser Fraktion wurden 2,5 g in 4,4 g einer Mischung aus 2,8 g Chloroform und 1,6 g Petroläther aufgenommen, aus der nach Abkühlung das reine (I) vom Festpunkt f = 75 bis 76°C auskristallisierte.

**Patentansprüche**

1. Verfahren zur Herstellung von 2,5-Dimethyl-4-hydroxy-2,3-dihydrofuran-3-on, *dadurch gekennzeichnet,* daß man

a) Hex-3-en-2,5-diol (V) in an sich bekannter Weise in flüssiger Phase mit Wasserstoffperoxid in Gegenwart eines Oxids eines Metalles der 4. bis 8. Nebengruppe oder einer Heteropolysäure eines der Säure bildenden Elemente der Gruppe VI des Periodensystems der Elemente bei einem pH-Wert von 3 bis 7, vorzugsweise 4 bis 6 zum 3,4-Epoxy-hexan-2,5-diol (II) epoxidiert,

b) das erhaltene 3,4-Epoxy-hexan-2,5-diol in an sich bekannter Weise mittels katalytischer Mengen einer Säure bei Temperaturen von 40 bis 280°C unter Spaltung des Epoxidringes und anschließendem Ringschluß in 2,5-Dimethyl-3,4-dihydroxy-tetrahydrofuran (III) überführt,

c) das erhaltene 2,5-Dimethyl-3,4-dihydroxy-tetrahydrofuran mittels Sauerstoff oder einem Sauerstoff enthaltenden Gas bei Temperaturen von 400 bis 700°C an einem Silber- oder Kupfer-kontakt zu 2,5-Dimethyl-4-hydroxy-tetrahydrofuran-3-on (IV) dehydriert und

d) das erhaltene 2,5-Dimethyl-4-hydroxy-tetrahydrofuran-3-on in konzentriert essigsaurer Lösung mit Wismutoxid zu dem 2,5-Dimethyl-4-hydroxy-2,3-dihydrofuran-3-on oxidiert.

2. 2,5-Dimethyl-3,4-dihydroxy-tetrahydrofuran.

3. 2,5-Dimethyl-4-hydroxy-tetrahydrofuran-3-on.

**Revendications**

1. Procédé pour la préparation de 2,5-diméthyl-4-hydroxy-2,3-dihydroxyruranne-3-one, caractérisé en ce que

a) l'on époxyde l'hex-3-ène-2,5-diol (V) de façon connue en soi, en phase liquide, avec du peroxyde d'hydrogène en présence d'un oxyde d'un métal des 4ème à 8ème groupes auxiliaires ou d'un hétéropolyacide de l'un des éléments formateur d'acide du groupe VI du système périodique des éléments, à un pH de 3 à 7, de préférence de 4 à 6, pour obtenir le 3,4-époxy-hexane-2,5-diol (II);

b) on transforme en 2,5-diméthyl-3,4-dihydroxy-tétrahydrofuranne (III) le 3,4-époxy-hexane-2,5-diol obtenu, de façon connue en soi, au moyen de quantités catalytiques d'un acide, à une température de 40 à 280°C, avec ouverture du noyau époxyde, puis cyclisation;

c) on déshydrogène le 2,5-diméthyl-3,4-dihydroxy-tétrahydrofuranne obtenu au moyen d'oxygène ou d'un gaz contenant de l'oxygène, à une température de 400 à 700°C, en présence d'un catalyseur à l'argent ou au cuivre, pour obtenir la 2,5-diméthyl-4-hydroxyfuranne-3-one; et

d) on oxyde la 2,5-diméthyl-4-hydroxy-tétrahydrofuranne-3-one obtenue en solution acétique concentrée, avec de l'oxyde de bismuth, pour obtenir la 2,5-diméthyl-4-hydroxy-2,3-dihydroxyfuranne-3-one.

2. 2,5-diméthyl-3,4-dihydroxy-tétrahydrofuranne.

3. 2,5-diméthyl-4-hydroxy-tétrahydrofuranne-3-one.

**Claims**

1. A process for the preparation of 2,5-dimethyl-4-hydroxy-2,3-dihydrofuran-3-one, wherein

(a) hex-3-ene-2,5-diol (V) is epoxidized in a conventional manner in the liquid phase, by means of hydrogen peroxide in the presence of an oxide of a metal of subgroups IV to VIII or of a heteropolyacid of one of the acid-forming elements of group VI of the periodic table of the elements, at a pH of from 3 to 7, preferably from 4 to 6, to give 3,4-epoxy-hexane-2,5-diol (II),

(b) the resulting 3,4-epoxy-hexane-2,5-diol is converted at 40 to 280°C in a conventional manner, by means of catalytic amounts of an acid, by cleavage of the epoxide ring and subsequent cyclization, into 2,5-dimethyl-3,4-dihydroxy-tetrahydrofuran (III),

(c) the resulting 2,5-dimethyl-3,4-dihydroxy-tetrahydrofuran is dehydrogenated by means of oxygen or

an oxygen-containing gas over a silver or copper catalyst, at from 400 to 700°C, to give 2,5-dimethyl-4-hydroxy-tetrahydrofuran-3-one (IV), and

(d) the resulting 2,5-dimethyl-4-hydroxy-tetrahydrofuran-3-one is oxidized with bismuth oxide, in concentrated acetic acid solution, to give 2,5-dimethyl-4-hydroxy-2,3-dihydrofuran-3-one.

2. 2,5-Dimethyl-3,4-dihydroxy-tetrahydrofuran.

3. 2,5-Dimethyl-4-hydroxy-tetrahydrofuran-3-one.